Europäisches Patentamt

(19) European Patent Office

Office européen des brevets

(11) Veröffentlichungsnummer : **0 080 095**
**B1**

(12) **EUROPÄISCHE PATENTSCHRIFT**

(45) Veröffentlichungstag der Patentschrift :
28.05.86

(51) Int. Cl.⁴ : **C 12 P  7/28**

(21) Anmeldenummer : 82110258.9

(22) Anmeldetag : 08.11.82

(54) Verfahren zur Vergärung von kohlehydrat- und phosphathaltigen flüssigen Medien.

(30) Priorität : 20.11.81 DE 3146084

(43) Veröffentlichungstag der Anmeldung :
01.06.83 Patentblatt 83/22

(45) Bekanntmachung des Hinweises auf die Patenterteilung : 28.05.86 Patentblatt 86/22

(84) Benannte Vertragsstaaten :
AT BE CH DE FR GB IT LI LU NL SE

(56) Entgegenhaltungen :
DE-C-   629 694
US-A- 2 107 262
US-A- 2 481 263
CHEMICAL ABSTRACTS, Band 60, Nr. 5, 2. März 1964, Spalten 6186h, 6187a, Columbus, Ohio, USA
H.J. REHM: "Industrielle Mikrobiologie, 2. Auflage, 1980, Kapitel 20, Seiten 291-305, Srpinger Verlag, Berlin, DE. "Butanol-Aceton und weitere primäre Produkte aus Clostridien"
EUROPEAN JOURNAL OF APPLIED MICROBIOLOGY AND BIOTECHNOLOGY, Nr. 15, 1982, Seiten 201-205, Springer Verlag, Berlin, DE. H. BAHL et al.: "Continuous production of acetone and butanol by Clostridium acetobutylicum in a two-stage phosphate limited chemostat"
Die Akte enthält technische Angaben, die nach dem Eingang der Anmeldung eingereicht wurden und die nicht in dieser Patentschrift enthalten sind.

(73) Patentinhaber : Gesellschaft für Biotechnologische Forschung mbH (GBF)
Mascheroder Weg 1
D-3300 Braunschweig-Stöckheim (DE)

(72) Erfinder : Andersch, Wolfram, Dipl.-Biol.
Eschenweg 12
D-3200 Hildesheim (DE)
Erfinder : Bahl, Hubert, Dipl.-Biol.
Fichtenweg 14
D-4572 Essen-Oldenburg (DE)
Erfinder : Gottschalk, Gerhard, Prof. Dr.
Johann-Wolf-Strasse 30a
D-3412 Nörten-Hardenberg (DE)

(74) Vertreter : Boeters, Hans Dietrich, Dr. et al
Boeters, Bauer & Partner Thomas-Wimmer-Ring 14
D-8000 München 22 (DE)

**Beschreibung**

Die Erfindung betrifft ein Verfahren zur Vergärung von kohlehydrat- und phosphathaltigen flüssigen Medien mit Hilfe von Butanol, Aceton und/oder Ethanol als Gärungsprodukte bildenden Bakterien.

Verfahren zur Vergärung von kohlehydrat- und phosphathaltigen flüssigen Medien mit Hilfe von Butanol, Aceton und/oder Ethanol als Gärungsprodukte bildenden Bakterien sind seit Jahrzehnten bekannt. Dazu sei beispielsweise auf folgenden Stand der Technik verwiesen :

DE-PS 612 535, 629 679, 635 572, 659 389, 683 198, 700 493, 824 932, 920 724, 941 184 und 941 185.

GB-PS 4845, 15203, 15204, 319 079, 319 642 und 496 137.

JA-PS 61.5797.

US-PS 1 315 585, 1 510 526, 1 537 597, 1 538 516, 1 545 694, 1 668 814, 1 822 139, 1 908 361, 1 911 411, 1 913 164, 1 992 921, 2 050 219, 2 089 522, 2 089 562, 2 107 262, 2 113 471, 2 113 472, 2 123 078, 2 132 039, 2 132 358, 2 139 108, 2 139 111, 2 198 104, 2 202 161, 2 260 126, 2 326 425, 2 368 074, 2 369 680, 2 377 197, 2 398 837, 2 433 232, 2 439 791, 2 481 263 und 2 945 786.

Ferner US-Patent Quarterly, 12 (1932 a) 47-57 und 15 (1932 b) 237.

Verwendbare Bakterien sind dem Fachmann geläufig und lassen sich beispielsweise den angeführten Druckschriften entnehmen. Es handelt sich insbesondere um Arten und Stämme der Gattung Clostridium, wie Clostridium acetobutylicum, saccharobutylacetonicum, saccharoacetobutylicum (beispielsweise saccharoacetobutylicum-alpha, -beta oder -gamma), saccharoperbutylacetonicum, invertoacetobutylicum, propylbutylicum, madisonii, tetrylium und saccharobutylacetonicumliquefaciens (beispielsweise saccharobutylacetonicumliquefaciens-alpha, -beta, -gamma oder -delta).

Ferner kann man beispielsweise der US-PS 2 107 262 entnehmen, daß das bekannte Verfahren in Gegenwart von löslichem mineralischen Phosphat in einem Konzentrationsbereich von 0,01 bis 0,15 Gewichtsprozent durchgeführt wird (Zeilen 48-50 auf Seite 1 links der Druckschrift). Die Menge an zuzugebendem Phosphat wird durch Vorversuche bestimmt und hängt von der Menge des mit dem Gärmedium eingebrachten löslichen mineralischen Phosphats ab (Zeilen 18-27 und 37-39 auf Seite 1 rechts der Druckschrift).

Beim Stand der Technik haben jedoch die erzielbaren Ausbeuten an den gewünschten Gärungsprodukten Butanol, Aceton und Ethanol noch nicht befriedigt, wobei nach dem bekannten Stand der Technik auch unbefriedigenderweise nicht kontinuierlich gearbeitet wird.

Bei der Erfindung zugrundeliegenden Versuchen wurde festgestellt, daß sich eine kontinuierliche Vergärung dann durchführen läßt, wenn man unter Phosphatlimitierung arbeitet. Demgemäß betrifft die Erfindung mit einer Ausführungsform ein Verfahren zur Vergärung von kohlehydrat- und phosphathaltigen Medien mit Hilfe von Butanol, Aceton und/oder Ethanol als Gärungsprodukte bildenden Bakterien, wobei das Verfahren dadurch gekennzeichnet ist, daß man das Verfahren kontinuierlich unter bezüglich des gelösten anorganischen oder des im Medium enthaltenen gesamten Phosphats limitierten Bedingungen durchführt.

Viele zuckerhaltige Medien weisen nun Konzentrationen an gelöstem anorganischen Phosphat oder an gesamtem Phosphat auf, die oberhalb des für eine Phosphatlimitierung praktikablen Bereichs liegen. Derartige Medien werden daher erst dann in das Verfahren eingesetzt, wenn sie einer Phosphatfällung bis zu einer der Phosphatlimitierung entsprechenden Konzentration an anorganischem oder gesamtem Phosphat unterworfen worden sind.

Der mit der Durchführung von kontinuierlichen Verfahren unter Verwendung von Mikroorganismen vertraute Fachmann ist ohne weiteres in der Lage, den für eine Phosphatlimitierung geeigneten Konzentrationsbereich zu ermitteln. Dafür stehen dem Fachmann folgende Rahmenbedingungen zur Verfügung. Wird eine für eine Phosphatlimitierung zu hohe Phosphatkonzentration gewählt, so wird das eingespeiste Phosphat nicht verbraucht. Bei zu geringer Phosphatkonzentration fällt die optische Dichte ab, und im Extremfall kann das Wachstum ausbleiben. Lediglich beispielhaft sei für eine Phosphatlimitierung eine Konzentration an anorganischem oder an gesamtem Phosphat im Bereich von 1,0 bis 0,4 und insbesondere von 0,75 bis 0,5 mmolar genannt.

Erfindungsgemäß wurde ferner festgestellt, daß sich die Bildung der erwünschten Gärungsprodukte Butanol, Aceton und/oder Ethanol in einer nachgeschalteten zweiten Verfahrensstufe fortsetzen läßt. Diese kann wahlweise gleichfalls kontinuierlich oder alternativ chargenweise durchgeführt werden.

Rahmenbedingungen sind darin zu sehen, daß man in der ersten Stufe (Wachstumsstufe) eine derartige Durchflußrate wählt, daß die Bakterienkonzentration konstant hoch bleibt, d. h. daß sich ein Fließgleichgewicht (steady state) einstellt und daß bereits ein Teil des Substrats zu Butanol, Aceton und/oder Ethanol umgesetzt wird. Wählt man eine zu hohe Durchflußrate, so werden mehr Zellen ausgetragen als gebildet. Bei zu geringer Durchflußrate kann das Zellwachstum ggf. periodisch eingestellt werden. Anliegen der zweiten Stufe ist es, eine möglichst hohe Ausbeute an den gewünschten Gärungsprodukten in möglichst kurzer Zeit zu erzielen. Die Durchflußrate ist entsprechend einzustellen. Lediglich beispielhaft seien folgende speziellen Bedingungen angegeben :

1. Stufe

Durchflußrate 0,04 bis 0,20, insbesondere 0,06 bis 0,15 h$^{-1}$

pH 4,0 bis 5,0, insbesondere 4,0 bis 4,7, vorzugsweise 4,3 bis 4,5

Temperatur 30 bis 40, insbesondere 30 bis 37 °C

2. Stufe

pH 3,0 bis 5,0 insbesondere 4,0 bis 4,7, vorzugsweise 4,3 bis 4,5

Temperatur 25 bis 39, insbesondere 30 bis 36, vorzugsweise 32 bis 35 °C

Gemäß einer anderen Ausführungsform der Erfindung wird ein chargenweises Verfahren zur Vergärung von kohlehydrat- und phosphathaltigen flüssigen Medien mit Hilfe von Butanol, Aceton und/oder Ethanol als Gärungsprodukte bildenden Bakterien vorgeschlagen, wobei das Verfahren dadurch gekennzeichnet ist, daß man in das Verfahren

(a) ein kohlehydrathaltiges flüssiges Medium mit einer Konzentration an gelöstem anorganischen oder im Medium enthaltenen gesamten Phosphat im Bereich von 1,0 bis 0,4, insbesondere 0,75 bis 0,5 mmolar einsetzt,

(b) nach beendeter Wachstumsphase bei einem pH-Wert im Bereich 5 von 3,5 bis 5,0 und

(c) nach beendeter Wachstumsphase bei einer reduzierten Temperatur arbeitet.

Rahmenbedingungen für das chargenweise Verfahren bestehen darin, daß man die Wachstumsphase bei einer etwas höheren Temperatur als die anschließende Gärungsphase durchführt. Die Gärungsphase beginnt bei Phosphataufbrauch. Beim chargenweisen Verfahren sollte der pH-Wert nicht unter 3,5 und insbesondere nicht unter 4,0 liegen.

Auch für diese Ausführungsform gilt, daß viele kohlehydrathaltige vergärbare Medien Konzentrationen an anorganischem oder an gesamtem Phosphat besitzen, die oberhalb des angegebenen Bereichs von 1,0 bis 0,4 mmolar liegen. Bevor derartige Medien in das erfindungsgemäße chargenweise Verfahren eingesetzt werden, unterwirft man sie einer Phosphatfällung bis zu einer Konzentration an anorganischem oder an gesamtem Phosphat im Bereich von 1,0 bis 0,4 und insbesondere 0,75 bis 0,5 mmolar.

Nachstehend wird die Erfindung durch vier Figuren und drei Beispiele näher erläutert. Der in den Beispielen verwendete Mikroorganismus Clostridium acetobutylicum liegt bei der Deutschen Sammlung von Mikroorganismen/Göttingen unter der Nr. 1731.

A) Kontinuierliches einstufiges Verfahren

Beispiel 1

Es wurde ein 250-ml-Kleinfermenter (Eigenkonstruktion in Zusammenarbeit mit der Fa. P. Ochs) verwendet, der mit einem Einleitungsrohr für Stickstoff und Nährlösung, einer Laugedosiereinrichtung, einem pH-Meßgerät, einem Magnetrührer, einem Heizmantel und einem Überlaufrohr für Gas und Zellsuspension ausgestattet war. Es wurde ein wässeriges Medium folgender Zusammensetzung verwendet:

Gärmedium

Angaben pro Liter:

| | |
|---|---|
| $KH_2PO_4$ | 0.1 g |
| | 0.735 mMol |
| $(NH_4)_2SO_4$ | 2.0 g |
| $FeSO_4 \times 7\ H_2O$ | 15 mg |
| Glucose $\times$ $H_2O$ | 60 g |
| Salzlösung | 10 ml |
| Vitaminlösung | 1 ml |
| $Na_2S_2O_4$ | 35 mg |

Vitaminlösung

Angaben pro 100 ml:

| | |
|---|---|
| Thiamindichlorid | 200 mg |
| 4-Aminobenzoesäure | 200 mg |
| D(+)-Biotin | 10 mg |

Salzlösung

Angaben pro Liter:

| | |
|---|---|
| $MgSO_4 \times 7\ H_2O$ | 10 g |
| NaCl | 1 g |
| $Na_2MoO_4 \times 2\ H_2O$ | 1 g |
| $CaCl_2 \times 2\ H_2O$ | 1 g |
| $MnSO_4 \times H_2O$ | 1.5 g |

# 0 080 095

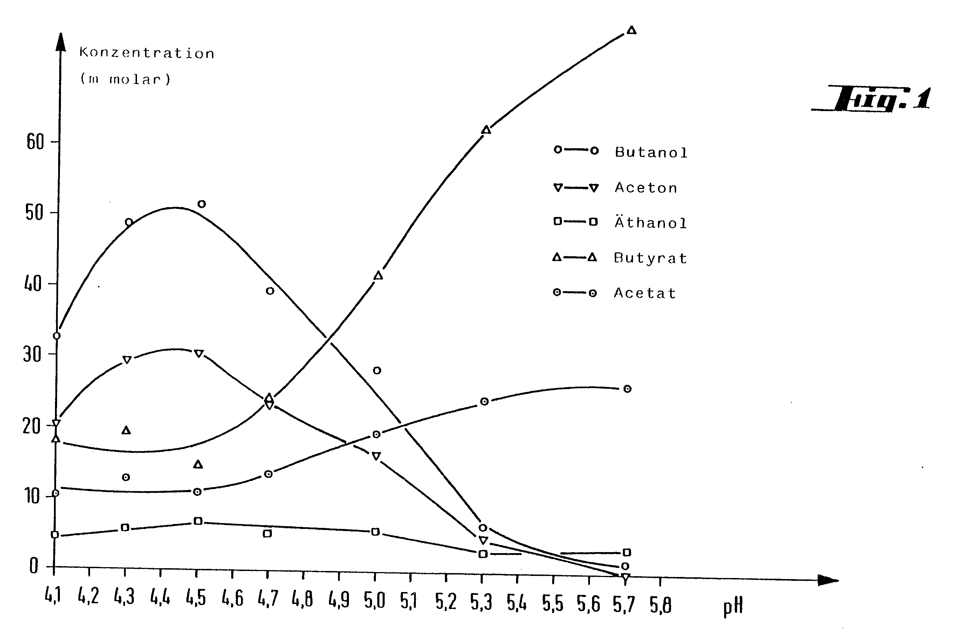

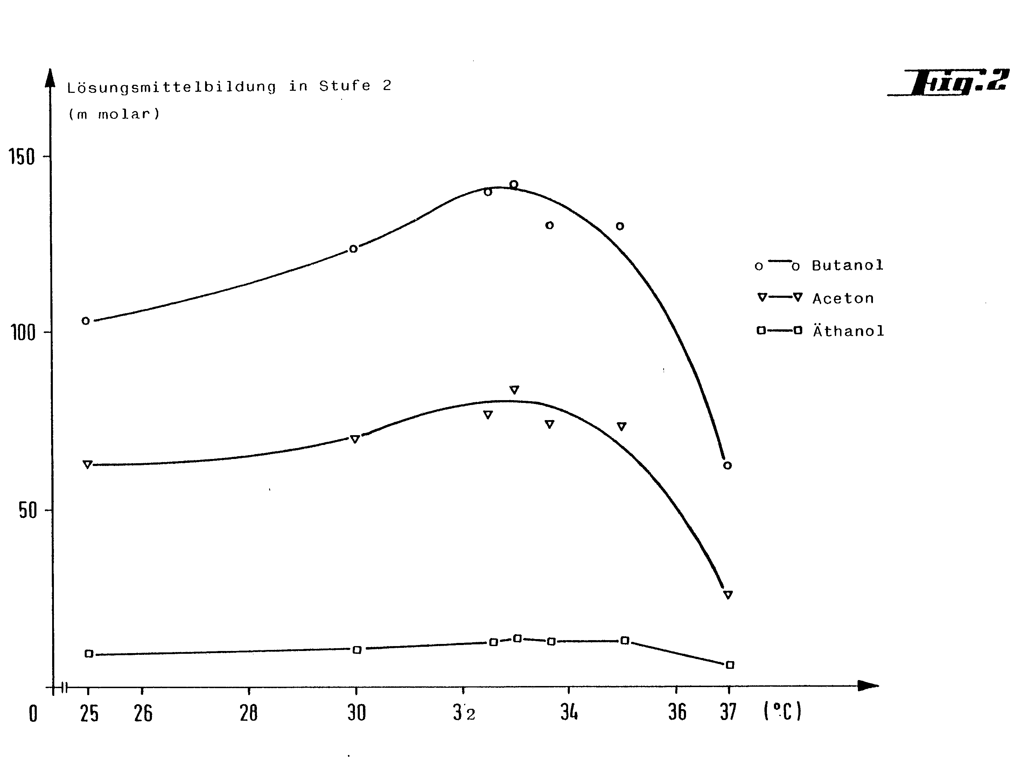

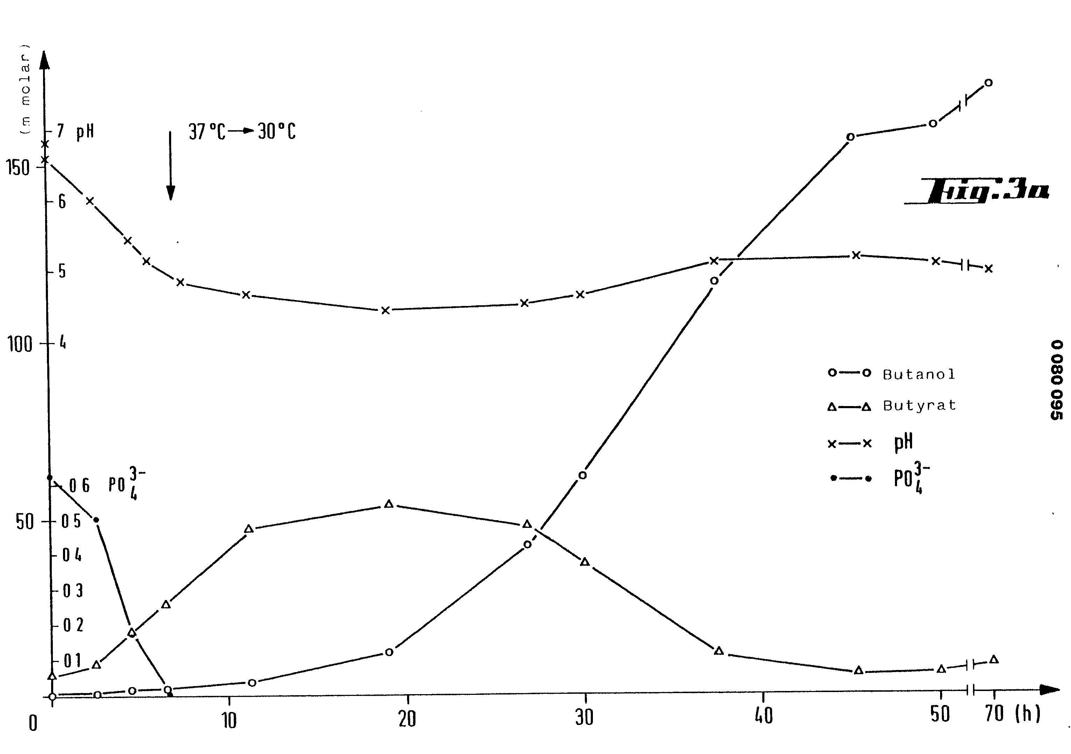

Untersucht wurden die Produktkonzentrationen unter Phosphatlimitierung bei verschiedenen pH-Werten und konstanter Durchflußrate (D) von 0.127 $h^{-1}$. Die Versuchsergebnisse sind Figur 1 und Tabelle 1 zu entnehmen.

Tabelle 1

Produkt- und Subtratkonzentrationen (mmolar) unter Phosphatlimitierung bei verschiedenen pH-Werten

| pH | Glucose | $PO_4^{3-}$ | $NH_4^+$ | Butanol | Aceton | Äthanol | Butyrat | Acetat |
|-----|---------|-------|------|---------|--------|---------|---------|--------|
| 5.7 | 175 | 0.090 | 3.4 | 2.3 | 0.3 | 3.2 | 77.2 | 26.5 |
| 5.3 | 174 | 0.086 | 6.5 | 6.4 | 4.5 | 2.9 | 62.6 | 24.3 |
| 5.0 | 179 | 0.079 | 5.9 | 28.4 | 16.8 | 5.5 | 41.7 | 19.5 |
| 4.7 | 177 | 0.075 | 6.5 | 39.5 | 24.1 | 5.1 | 24.5 | 13.5 |
| 4.5 | 170 | 0.075 | 5.9 | 51.5 | 30.1 | 6.9 | 14.8 | 10.8 |
| 4.3 | 175 | 0.055 | 6.4 | 48.7 | 29.8 | 5.5 | 19.9 | 12.6 |
| 4.1 | 200 | 0.071 | 7.3 | 32.4 | 20.6 | 4.5 | 18.1 | 10.9 |

Figur 1 und Tabelle 1 zeigen, daß unter Phosphatlimitierung die steady-state-Konzentrationen (Fließgleichgewicht) an Butanol und Aceton mit fallendem pH zunehmen und zwischen pH 4.3 und 4.5 ein Maximum erreichen. Damit ist gezeigt, daß wachsende C.-acetobutylicum-Zellen unter den hier gefundenen Bedingungen Lösungsmittel bilden. Die Aceton-Butanol-Gärung kann demnach kontinuierlich durchgeführt werden.

Hinsichtlich des relativ geringen Subtratumsatzes bzw. des hohen Restzuckergehaltes wurde gefunden, daß sich die Lösungsmittelgärung in einer nachgeschalteten Stufe fortsetzen läßt.

B) Kontinuierliches zweistufiges Verfahren

Beispiel 2

Es wurde ein 2-l-Kleinfermenter (0.9 l Kulturvolumen) verwendet, der mit einem 5-l-Kleinfermenter (3 l Kulturvolumen ; Braun Biostat) verbunden war. Ausstattung (Rühren hier durch Schaufelräder) und Medium siehe Beispiel 1.

Kulturbedingungen in Stufe 1 :

pH  : 4.3
T   : 37 °C
D   : 0.125 $h^{-1}$
Upm : 75

Kulturbedingungen in Stufe 2 :

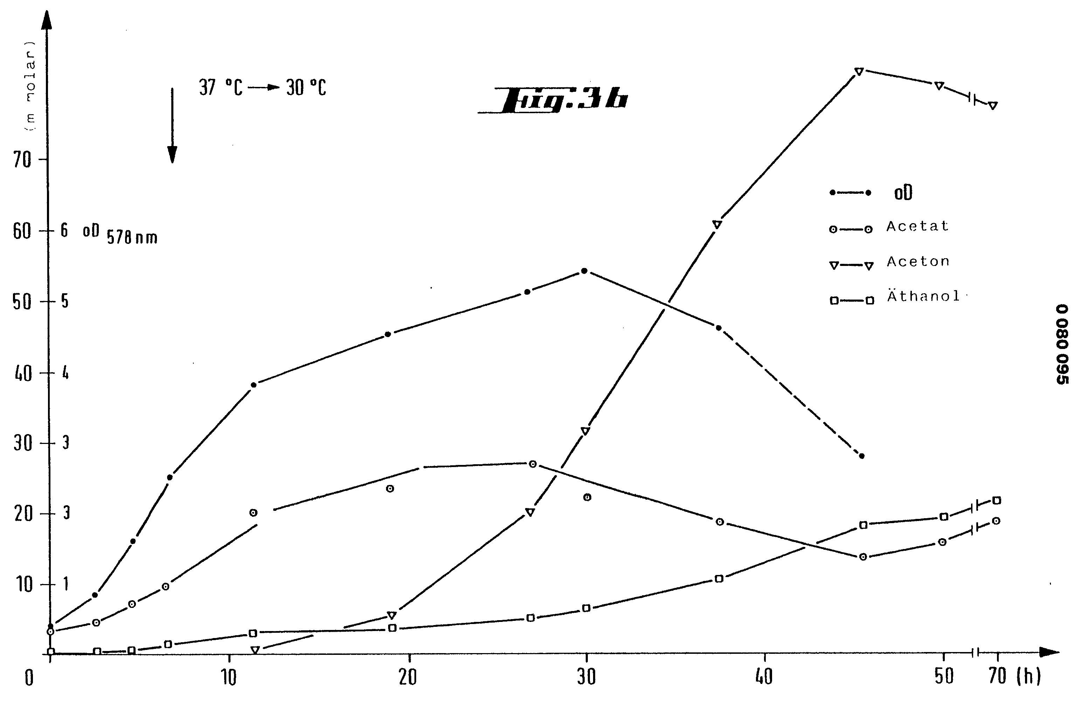

pH  : 4.3
T   : variabel, siehe Figur 2
D   : 0.037 5 $h^{-1}$
Upm : 100

Die Substrat- und Produktkonzentrationen der Stufen 1 und 2 unter den angegebenen Bedingungen sind in Tabelle 2 dargestellt. Figur 2 zeigt die Abhängigkeit der Lösungsmittelbildung in Stufe 2 von der Temperatur. Die Säurebildung veränderte sich im getesteten Temperaturbereich nur unwesentlich. Die in Tabelle 2 und Figur 2 angegebenen Daten zeigen, daß unter Phophatlimitierung in einer zweistufigen Anlage die Aceton-Butanolgärung mit hohem Substratumsatz und hoher Ausbeute an den gewünschten Produkten kontinuierlich möglich ist. Die hier gewählten Durchflußraten sind lediglich beispielhaft. In Stufe 1 läßt sich D im Bereich von 0,04 bis 0,20 $h^{-1}$ variieren, wobei die Produktkonzentration mit sinkender Durchflußrate zunimmt. In Stufe 2 muß die Durchflußrate so gewählt werden, daß die entsprechend lange Verweildauer im Fermenter für einen hohen Substratumsatz ausreicht. Für C. acetobutylicum DSM 1731 wurde dabei folgende Regel gefunden : Je langsamer die Durchflußrate in der Stufe 2 ist, umso niedriger ist die optimale Temperatur.

Tabelle 2

Substrat- und Produktkonzentration in Stufen 1 und 2 der kontinuierlichen Anlage

| | Stufe 1<br>T : 37°C<br>D : 0.125 h$^{-1}$ | | Stufe 2<br>T : 33°C<br>D : 0.0375 h$^{-1}$ | |
|---|---|---|---|---|
| Butanol | 41.5 | mmolar | 142 | mmolar |
| Aceton | 25.4 | " | 84 | " |
| Äthanol | 5 | " | 13 | " |
| Butyrat | 19.4 | " | 11.5 | " |
| Acetat | 12 | " | 15 | " |
| Acetoin | 0.8 | " | 2.3 | " |
| Glucose | 205 | " | 30.5 | " |
| PO$_4^{3-}$ | 0.075 | " | 0.01 | " |
| Substratverbrauch | 33 % | | 90 % | |
| davon Lösungsmittel | 69 % | | 86 % | |

Die höhere Temperatur (37°) in Stufe 1 (Wachstumsphase) verglichen mit Stufe 2 (33 °C, Gärungsphase) erwies sich als günstig : Sie ermöglichte schnelleres Wachstum, zum anderen war die Butanolkonzentration doppelt so hoch wie zum Beispiel bei 30 °C. Medien mit höherer Substratkonzentration lassen sich bei Wahl geeigneter Durchflußraten ebenfalls in hoher Ausbeute zu Lösungsmitteln umsetzen.

Es ist nicht notwendig, die 2. Stufe kontinuierlich zu betreiben, sondern auch möglich, den Fermenterausfluß der 1. Stufe aufzufangen, z. B. bei 30 °C und pH 4.3 zu incubieren und dann zu ernten, wenn der Restzucker zu Lösungsmitteln vergoren ist (Dauer 1.5 bis 2 Tage).

C) Batchverfahren

Beispiel 3

Das Prinzip der Phosphatlimitierung unter den oben angegebenen Bedingungen in kontinuierlicher Kultur, das eine kontrollierbare Aceton-Butanolgärung beinhaltet, ließ sich auch auf das Batchverfahren übertragen.

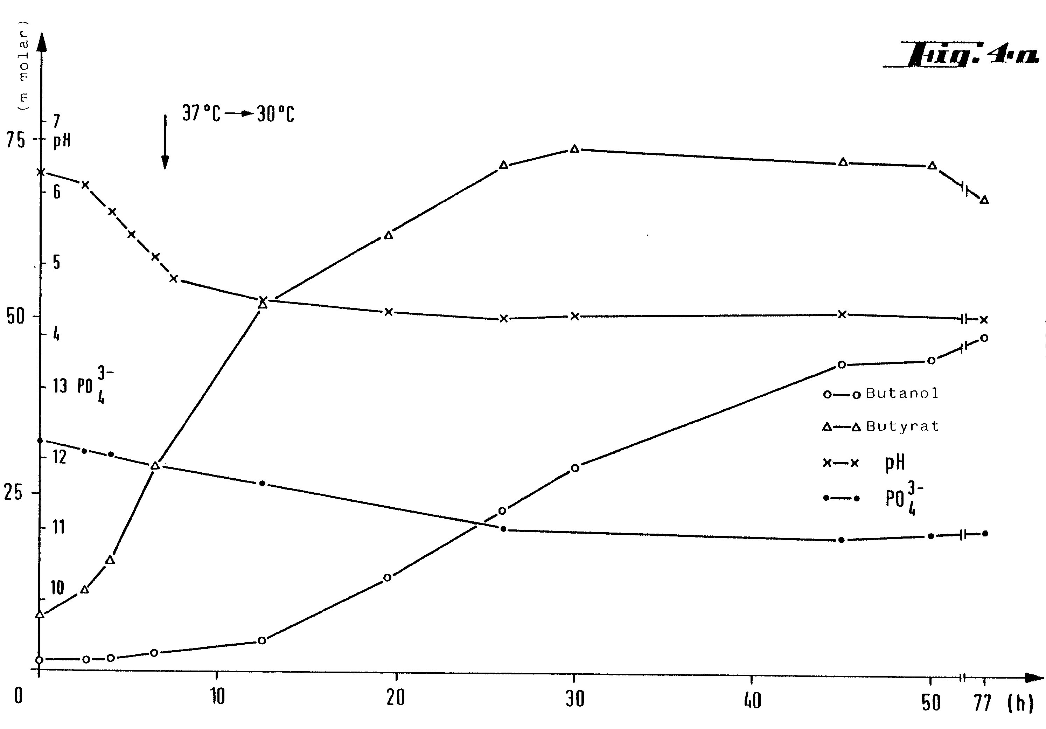

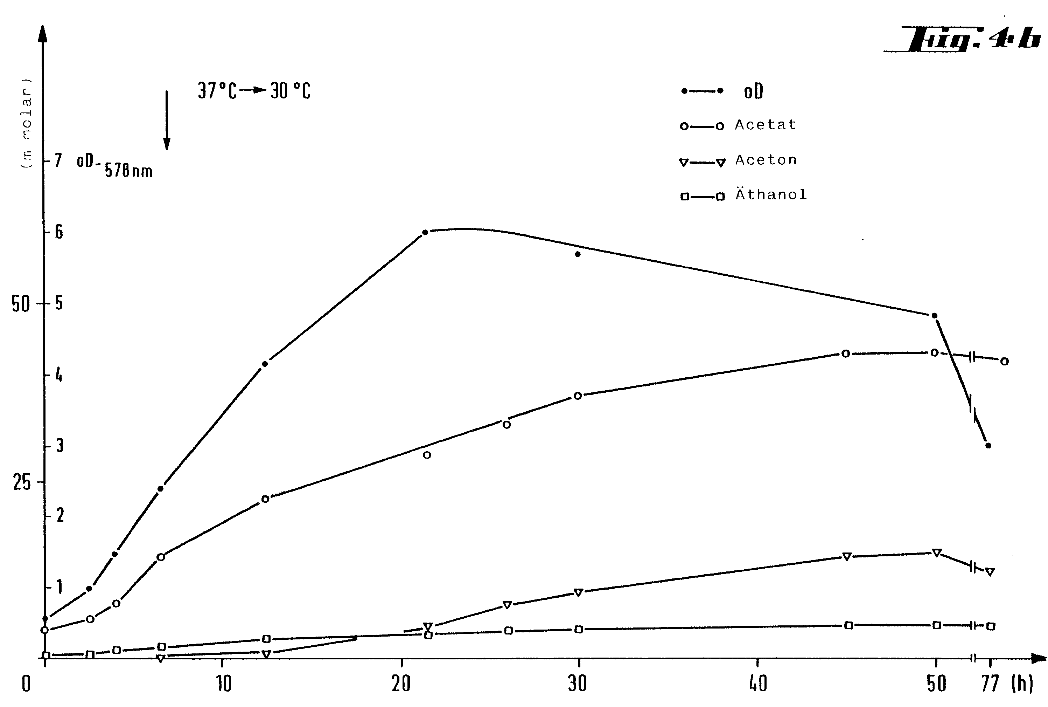

Medium und Fermenter wurden wie im Beispiel 2 gewählt. Als Vorkultur dienten Zellen aus Stufe 1. Entsprechend den Erkenntnissen aus der kontinuierlichen Kultur wurde folgende Versuchsführung eingehalten : In der Wachstumsphase wurde bei 37 °C incubiert. Zu diesem Zeitpunkt ist noch Phosphat im Medium vorhanden. Während dieser Zeit wurde durch Titration mit KOH ein zu schnelles Absinken des pH-Wertes verhindert, wodurch das Wachstum behindert worden wäre. Zu Beginn der Gärungsphase (pH unter 5, kein Phosphat im Medium mehr nachweisbar) wurde die Temperatur auf 30 °C eingestellt. Unter diesen Bedingungen zeigt C. acetobutylicum DSM 1731 in Batchkultur den in Figur 3 dargestellten Gärungsverlauf. Figur 3 zeigt, daß nach Phosphatverbrauch und Absinken des pH-Wertes unter 5 C. acetobutylicum von Säure- auf Lösungsmittelgärung umschaltet und die 6 %ige Zuckerlösung mit hoher Ausbeute zu Lösungsmitteln vergärt. In Figur 4 ist der Verlauf einer Batchkultur unter sonst gleichen Bedingungen, aber mit 20-facher Phosphatkonzentration gezeigt. Hier konnte kein Umschalten der Gärung beobachtet werden. Zwar setzte bei pH-Werten unter 5 auch Lösungsmittelbildung ein, jedoch wurde die gebildete Säure nicht wieder abgebaut, sondern deren Konzentration stieg weiter an. Dieses führte zu einer niedrigen Ausbeute an Lösungsmittel, zum anderen war der Substratumsatz geringer (siehe Tabelle 3).

Tabelle 3

Substrat- und Produktkonzentration in Batchkulturen unter Phosphatmangel und -überschuß nach 3 Tagen

|  | Phosphatmangel | | Phosphatüberschuß | |
|---|---|---|---|---|
| Butanol | 175 | mmolar | 47 | mmolar |
| Aceton | $77^x$ | " | $12^{xx}$ | " |
| Äthanol | 21.5 | " | 4.5 | " |
| Butyrat | 8.5 | " | 67.5 | " |
| Acetat | 18 | " | 42 | " |
| Acetoin | 9 | " | 7.5 | " |
| Glucose | 0 | " | 129 | " |
| $PO_4^{3-}$ | 0 | " | 11 | " |
| Substratverbrauch | 100 % | | 57 % | |
| davon Lösungsmittel | 87.5 % | | 36 % | |

[x]) nach zwei Tagen 84 mmolar
[xx]) nach zwei Tagen 15 mmolar

**Patentansprüche**

1. Verfahren zur Vergärung von kohlehydrat- und phosphathaltigen Medien mit Hilfe von Butanol, Aceton und/oder Ethanol als Gärungsprodukte bildenden Bakterien, dadurch gekennzeichnet, daß man das Verfahren kontinuierlich unter bezüglich des gelösten anorganischen oder des im Medium enthaltenen gesamten Phosphats limitierten Bedingungen durchführt.

2. Verfahren nach Anspruch 1, dadurch gekennzeichnet, daß man in das Verfahren ein kohlehydrathaltiges flüssiges Medium einsetzt, das einer Phosphatfällung bis zu einer der Phosphatlimitierung entsprechenden Konzentration an anorganischem oder gesamtem Phosphat unterworfen worden ist.

3. Verfahren nach Anspruch 1, dadurch gekennzeichnet, daß man in das Verfahren ein kohlehydrathaltiges Medium einsetzt, dem Phosphat bis zu einer der Phosphatlimitierung entsprechenden Konzentration zugesetzt wird.

4. Verfahren nach Anspruch 1, 2 oder 3, dadurch gekennzeichnet, daß man in das Verfahren ein kohlehydrathaltiges flüssiges Medium mit einer Konzentration an anorganischem oder gesamtem Phosphat im Bereich von 1,0 bis 0,4 und insbesondere 0,75 bis 0,5 mmolar einsetzt.

5. Verfahren nach einem der vorhergehenden Ansprüche dadurch gekennzeichnet, daß man das Verfahren zweistufig oder mehrstufig durchführt.

6. Verfahren nach Anspruch 5, dadurch gekennzeichnet, daß man die zweite Verfahrensstufe kontinuierlich oder chargenweise durchführt.

7. Verfahren nach Anspruch 4 oder 5, dadurch gekennzeichnet, daß man das Verfahren unter folgenden Bedingungen durchführt:

1. Stufe:
Durchflußrate 0,04 bis 0,20 ; insbesondere 0,06 bis 0,15 $h^{-1}$
pH 4,0 bis 5,0 ; insbesondere 4,0 bis 4,7 ; vorzugsweise 4,3 bis 4,5
Temperatur 30 bis 40 ; insbesondere 30 bis 37 °C

2. Stufe:
pH 3,0 bis 5,0 ; insbesondere 4,0 bis 4,7 ; vorzugsweise 4,3 bis 4,5
Temperatur 25 bis 39 ; insbesondere 30 bis 36 ; vorzugsweise 32 bis 35 °C

8. Chargenweises Verfahren zur Vergärung von kohlehydrat- und phosphathaltigen Medien mit Hilfe von Butanol, Aceton und/oder Ethanol als Gärungsprodukte bildenden Bakterien, dadurch gekennzeichnet, daß man das Verfahren unter bezüglich des gelösten anorganischen oder des im Medium enthaltenen gesamten Phosphats limitierten Bedingungen durchführt.

9. Verfahren nach Anspruch 8, dadurch gekennzeichnet, daß man in das Verfahren ein kohlehydrathaltiges Medium einsetzt, das einer Phosphatfällung bis zu einer der Phosphatlimitierung entsprechenden Konzentration an anorganischem oder gesamtem Phosphat unterworfen worden ist.

6

10. Verfahren nach Anspruch 8, dadurch gekennzeichnet, daß man in das Verfahren ein kohlehydrathaltiges Medium einsetzt, dem Phosphat bis zu einer der Phosphatlimitierung entsprechenden Konzentration zugesetzt wird.

11. Verfahren nach Anspruch 8, 9 oder 10, dadurch gekennzeichnet, daß man in das Verfahren

(a) ein kohlehydrathaltiges Medium mit einer Konzentration an gelöstem anorganischen oder im Medium enthaltenen gesamten Phosphat im Bereich von 1,0 bis 0,4 und insbesondere von 0,75 bis 0,5 mmolar einsetzt,

(b) nach beendeter Wachstumsphase bei einem pH-Wert von 3,5 bis 5,0 und

(c) nach beendeter Wachstumsphase bei einer reduzierten Temperatur arbeitet.

## Claims

1. Process for fermenting carbohydrate- and phosphate-containing media with the aid of bacteria that form butanol, acetone and/or ethanol as the fermentation products, characterised in that the process is carried out continuously under conditions that are limited with regard to the dissolved inorganic phosphate or to the total phosphate contained in the medium.

2. Process according to claim 1, characterised in that there is used in the process a carbohydrate-containing liquid medium which has been subjected to a phosphate precipitation to give a concentration of inorganic phosphate or total phosphate that corresponds to the phosphate limitation.

3. Process according to claim 1, characterised in that there is used in the process a carbohydrate-containing medium to which phosphate is added to give a concentration corresponding to the phosphate limitation.

4. Process according to claim 1, 2 or 3, characterised in that there is used in the process a carbohydrate-containing liquid medium having a concentration of inorganic or total phosphate in the range of from 1.0 to 0.4 and especially from 0.75 to 0.5 mmolar.

5. Process according to any one of the preceding claims, characterised in that the process is carried out in two or more stages.

6. Process according to claim 5, characterised in that the second process stage is carried out continuously or batchwise.

7. Process according to claim 4 or 5, characterised in that the process is carried out under the following conditions :

1st stage :
flow rate from 0.04 to 0.20 ; especially from 0.06 to 0.15 $h^{-1}$
pH from 4.0 to 5.0 ; especially from 4.0 to 4.7 ; preferably from 4.3 to 4.5
temperature from 30 to 40 ; especially from 30 to 37 °C
2nd stage :
pH from 3.0 to 5.0 ; especially from 4.0 to 4.7 ; preferably from 4.3 to 4.5
temperature from 25 to 39 ; especially from 30 to 36 ; preferably from 32 to 35 °C.

8. Batchwise process for fermenting carbohydrate- and phosphate-containing media with the aid of bacteria that form butanol, acetone and/or ethanol as the fermentation products, characterised in that the process is carried out under conditions that are limited with regard to the dissolved inorganic phosphate or to the total phosphate contained in the medium.

9. Process according to claim 8, characterised in that there is used in the process a carbohydrate-containing medium which has been subjected to a phosphate precipitation to give a concentration of inorganic or total phosphate corresponding to the phosphate limitation.

10. Process according to claim 8, characterised in that there is used in the process a carbohydrate-containing medium to which phosphate is added to give a concentration corresponding to the phosphate limitation.

11. Process according to claim 8, 9 or 10, characterised in that there is used in the process

(a) a carbohydrate-containing medium having a concentration of dissolved inorganic phosphate or of total phosphate contained in the medium in the range of from 1.0 to 0.4 and especially 0.75 to 0.5 mmolar,

(b) after the growth phase is complete, the process is carried out at a pH value of from 3.5 to 5.0 and

(c) after the growth phase is complete, the process is carried out at a reduced temperature.

## Revendications

1. Procédé de fermentation de milieux contenant des glucides et des phosphates avec des bactéries qui donnent comme produits de fermentation du butanol, de l'acétone et/ou de l'éthanol, procédé caractérisé en ce qu'il est exécuté en continu dans des conditions limitées du phosphate minéral en solution ou du phosphate total que contient le milieu.

2. Procédé selon la revendication 1 caractérisé en ce que l'on utilise dans ce procédé un milieu liquide (contenant des glucides), dont on a fait précipiter les phosphates jusqu'à une concentration en phosphate minéral ou en phosphate total qui correspond à la limitation des phosphates.

3. Procédé selon la revendication 1 caractérisé en ce que l'on utilise dans ce procédé un milieu (contenant des glucides) auquel on ajoute du phosphate jusqu'à une concentration correspondant à la limitation des phosphates.

4. Procédé selon la revendication 1, 2 ou 3, caractérisé en ce que l'on utilise dans ce procédé un milieu liquide contenant des glucides à une concentration en phosphate minéral ou total 1,0 à 0,4 mmolaire, en particulier 0,75 à 0,5 mmolaire.

5. Procédé selon l'une quelconque des revendications précédentes, caractérisé en ce qu'il est exécuté en deux étapes ou plus.

6. Procédé selon la revendication 5 caractérisé en ce que la seconde étape se fait en continu ou en discontinu.

7. Procédé selon la revendication 4 ou 5, caractérisé en ce qu'il est conduit dans les conditions suivantes :

1ère étape :
Débit 0,04 à 0,20 ; en particulier 0,06 à 0,15 $h^{-1}$
pH 4,0 à 5,0 ; en particulier 4,0 à 4,7 ; de préférence 4,3 à 4,5
Température 30 à 40 ; en particulier 30 à 37 °C
2ème étape :
pH 3,0 à 5,0 ; en particulier 4,0 à 4,7 ; de préférence 4,3 à 4,5
Température 25 à 39 ; en particulier 30 à 36 ; de préférence 32 à 35 °C.

8. Procédé discontinu de fermentation de milieux contenant des glucides et des phosphates avec des bactéries qui donnent comme produits de fermentation du butanol, de l'acétone et/ou de l'éthanol, procédé caractérisé en ce qu'il est exécuté dans des conditions de limitation du phosphate minéral en solution ou du phosphate total que contient le milieu.

9. Procédé selon la revendication 8 caractérisé en ce que l'on utilise dans ce procédé un milieu (contenant des glucides), dont on a fait précipiter les phosphates jusqu'à une concentration en phosphate minéral ou total qui correspond à la limitation des phosphates.

10. Procédé selon la revendication 8 caractérisé en ce que l'on utilise dans ce procédé un milieu (contenant des glucides) auquel on ajoute du phosphate jusqu'à une concentration correspondant à la limitation des phosphates.

11. Procédé selon la revendication 8, 9 ou 10, caractérisé en ce que : on utilise dans ce procédé
a) un milieu contenant des glucides à une concentration en phosphate minéral dissous ou en phosphate total contenu dans le milieu 1,0 à 0,4 mmolaire, en particulier 0,75 à 0,5 mmolaire.
b) la phase de croissance (multiplication) terminée, on opère à un pH de 3,5 à 5,0, et
c) la phase de croissance terminée, on opère à une température réduite.

Fig. 1

Fig. 2

Lösungsmittelbildung in Stufe 2 (m molar)

o——o Butanol
▽——▽ Aceton
□——□ Äthanol

0 080 095

Fig.3a

Fig. 3b

Fig. 4·a

Fig. 4 b